# EUROPÄISCHE PATENTANMELDUNG

(11) **EP 1 281 370 A2**
(43) Veröffentlichungstag der Anmeldung: **05.02.2003**
(21) Anmeldenummer: 02016992.6
(22) Anmeldetag: 05.08.2002
(51) Int. Cl.: A61C 1/00, A61N 5/06

(54) **Bestrahlungsvorrichtung**

(30) Priorität: 03.08.2001 DE 10138071
(71) Anmelder: Edel, Susann, 82031 Grünwald (DE); Knott, Georg, 82377 Penzberg (DE)
(72) Erfinder: Edel, Susann, 82031 Grünwald (DE); Knott, Georg, 82377 Penzberg (DE)
(74) Vertreter: Banzer, Hans-Jörg, Dipl.-Ing.

(57) **Zusammenfassung**

Eine Bestrahlungsvorrichtung (10), welche insbesondere im Bereich Kosmetik zur Bleichung von Zähnen oder Haaren und im Bereich Medizin zur photodynamischen Diagnose oder Therapie und im Bereich Industrie zur Aushärtung von geeigneten Klebern oder Lacken geeignet ist, umfasst ein flächiges Leuchmittelsegment (11), welches in die große Lichteingangsfläche eines trichterförmigen Reflektors (13) einstrahlt, der diese Strahlung durch die besondere Form idealerweise ganz auf die sehr viel kleinere an der Lichtausgangsöffnung liegende Bestrahlungsfläche (12) bündelt. Vorzugsweise mit der Bedingung, dass der Öffnungswinkel (α) des Reflektortrichters (13) bei vorgegebener Größe von Leuchtmittelsegment (11) und Bestrahlungsfläche (12) mindestens so groß ist, dass der äusserste Randstrahl (14) noch auf die Bestrahlungsfläche (12) trifft, wird auf dieser Bestrahlungsfläche (12) durch den Strahlengang in dem so ausgeführten Reflektortrichter (13) eine vielfach höhere Bestrahlungsstärke erzielt, als mit dem Leuchtmittelsegment (11) selbst. Das Leuchtmittelsegment (11) weist bevorzugt eine Vielzahl von regelmäßig angeordneten Leuchtmitteln (15), insbesondere Leuchtdioden oder Laserdioden, auf, die vorzugsweise auf einer Leiterplatte (16) montiert sind.

## Beschreibung

Die vorliegende Erfindung betrifft eine Bestrahlungsvorrichtung zur Erzielung einer möglichst hohen Bestrahlungsstärke auf einer kleinen definierten Bestrahlungsfläche, insbesondere mit Leuchtdioden oder Laserdioden als Leuchtmittel, für unterschiedliche Anwendungen, insbesondere für medizinische, kosmetische oder industrielle Anwendungen.

In der Zahnmedizin wird seit vielen Jahren zum Bleichen von Zähnen das sogenannte Zahn-Bleaching Verfahren angewendet, bei dem der Zahn mit einem Bleichmittelgel das dafür geeignete chemische Substanzen enthält, umhüllt wird, welches dann mit Licht bestimmter Wellenlängen beispielsweise Laser-Licht oder mit gefiltertem Licht aus einer Gasentladungslampe bestrahlt wird, wobei das Licht den Bleichvorgang in der äußeren Zahnschicht auslöst. Der Nachteil der bisher angewendeten Laserlichtquellen ist der hohe Preis einerseits und das erhöhte Gefährdungspotential in der Anwendung durch den auch in größerer Entfernung noch hoch konzentrierten und damit für das menschliche Auge sehr gefährlichen Laserstrahl. Der Nachteil der bisher angewendeten Gasentladungslampen-Lichtquellen ist neben dem hohen Preis die Notwendigkeit, aus dem Gesamtspektrum dieser Lampen mittels optischen Filtern die für Menschen schädliche UV- und IR-Strahlung und die nicht gewünschte VIS-Strahlung auszufiltern, das Ableiten der dabei entstehenden Wärme und die Notwendigkeit mit einem Lichtleiter das Licht an das Objekt leiten zu müssen. Zusätzlich negativ kann sich die verbleibende Restwärme auswirken, da empfindliche Bereiche wie z.B. der Zahnnerv geschädigt und in Folge dessen Schmerzen ausgelöst werden können. Nicht bekannt war bisher eine derartige Bestrahlungsvorrichtung mit Leuchtdioden als Leuchtmittel, welche die oben genannten Nachteile nicht aufweist.

Aus der US 1,573,419 ist beispielsweise eine Bestrahlungsvorrichtung nach dem Oberbegriff des Anspruchs 1 bekannt. Diese Bestrahlungsvorrichtung umfasst eine Quecksilberlampe, welche Strahlung in eine Lichteintritts- bzw. Lichteingangsöffnung eines trichterförmigen Reflektors abstrahlt, wobei durch Reflexion an den Innenflächen des trichterförmigen Reflektors die Strahlung in einen Quarzkörper fokussiert wird, welcher in einer Lichtaustritts- bzw. Lichtausgangsöffnung des trichterförmigen Reflektors angeordnet ist. Bei dieser Druckschrift erfolgt jedoch eine im Prinzip willkürliche Abstrahlung von der Quecksilberlampe in den trichterförmigen Reflektor. Diese Tatsache sowie die Tatsache der Verwendung einer Quecksilberlampe machen eine Verwendung der in dieser Druckschrift beschriebenen Bestrahlungsvorrichtung beispielsweise für die oben beschriebenen Anwendungen unmöglich.

Der vorliegenden Erfindung liegt die Aufgabe zugrunde, eine Bestrahlungsvorrichtung bereitzustellen, welche mit möglichst einfachen Mitteln die Bestrahlung einer definierten Bestrahlungsfläche mit einer relativ hohen Bestrahlungsstärke ermöglicht, wobei zusätzlich eine größtmögliche Homogenität der Bestrahlung über die gewünschte Bestrahlungsfläche gewährleistet sein und eine maximale Sicherheit für den Anwender oder den Behandelten gegeben sein soll. Die Bestrahlungsvorrichtung soll darüber hinaus insbesondere für den Einsatz in der Zahnheilkunde für das Auslösen bestimmter Reaktionen chemischer Substanzen zum Bleichen von Zähnen, in der Kosmetik für das Bestrahlen von Hautunreinheiten oder das Bleichen von Haaren (ähnlich dem Bleichen von Zähnen) und in der Medizin für die photodynamische Therapie oder die photodynamische Diagnostik der Haut und von Schleimhäuten in Körperhöhlen (wie dem Mund) eingesetzt werden. Weitere Anwendungen ergeben sich in der Industrie für das Aushärten von Lacken, Klebstoffen, Füllmaterialien, Kunststoffen und das Aktivieren von mit Licht bestimmter Wellenlängen auszulösenden Reaktionen diverser chemischer Stoffe, wie beispielsweise das Auslösen des Polymerisationsvorgangs durch Licht oder UV-Strahlung oder Infrarot-Strahlung.

Die oben genannte Aufgabe wird erfindungsgemäß durch eine Bestrahlungsvorrichtung mit den Merkmalen des Anspruchs 1 gelöst. Die Unteransprüche definieren jeweils bevorzugte und vorteilhafte Ausführungsformen bzw. Anwendungen der vorliegenden Erfindung.

Die vorliegende Erfindung kann insbesondere auch für punktuelle Bestrahlungen im Rahmen der sogenannten Photodynamischen Therapie und Photodynamischen Diagnostik eingesetzt werden. Hier werden lichtempfindliche Substanzen, sog.
Photosensibililsatoren lokal oder systemisch in das menschliche Gewebe der Haut oder in das Gewebe bestimmter Organe eingebracht und anschließend mit dem Licht definierter Wellenlängenbereiche bestrahlt. Dabei wird vorzugsweise ein Photosensibilisator gewählt, der sich in krankhaft verändertem neoplastischem Gewebe selektiv und extrem stark anreichert. Bei der Photodynamischen Diagnostik wird dann durch Auswertung des in Folge der Bestrahlung des Photosensibilisators emittierten Fluoreszenzlichts mit einem Detektor ausgewertet. Auf diese Weise lassen sich Tumore oder unerwünschte Hautveränderungen zuverlässig erkennen und bildlich darstellen. Der Kontrast zwischen gesundem und krankhaft veränderten Gewebe ist neben der möglichst selektiven Anreicherung des verwendeten Photosensibilisators und dessen Lichtabsorption auch stark von der eingestrahlten Lichtleistung der verwendeten Anregungslichtquelle abhängig. Ein weiteres Kriterium ist das genaue Einhalten der definierten Wellenlängenbereiche der Anregungslichtquelle und die möglichst homogene Verteilung des Lichts über die zu bestrahlende Fläche.

Bei der Photodynamischen Therapie wird hingegen die Tatsache ausgenutzt, dass es zwischen dem Photosensibilisator und der biologischen Umgebung über eine komplexe Wechselwirkung zur Aktivierung von molekularem Sauerstoff in dem jeweils behandelten Gewebe kommt, wobei die auf diese Weise erzeugten Sauerstoffradikale hoch reaktiv sind und Biomoleküle in nächster Umgebung letal schädigen können. Der therapeutische Nutzen ist hierbei die selektive Zerstörung von bösartigem Tumorgewebe oder die Behandlung virusinduzierter Warzen und Entzündungsherde die durch Bakterien ausgelöst werden.

Die erfindungsgemäße Bestrahlungsvorrichtung umfasst eine flächig angeordnete Gruppe von Leuchtdioden oder Laserdioden in einem Leuchtmittelsegment deren Lichtemission durch einen als Trichter geformten Reflektor, der im rechten Winkel über diesen Lichtquellen angeordnet ist, so auf eine um ein Vielfaches kleinere Bestrahlungsfläche fokussiert wird, dass sich durch Überlappung des Lichts eine Vervielfachung der Bestrahlungsstärke im definierten Bestrahlungsfeld in Bezug auf die Bestrahlungsstärke der einzelnen Leuchtmittel ergibt.

Als Leuchtmittel werden dabei bevorzugt regelmäßige Anordnungen von Leuchtdioden oder Laserdioden verwendet, welche UV-Strahlung, IR-Strahlung oder sichtbares Licht emittieren und den gewünschten Strahlungsverlauf des jeweiligen Leuchtmittelsegments dadurch erzeugen, dass der Abstrahlwinkel der einzelnen Leuchtmittel auf einen bestimmten Wert, insbesondere < 30 Grad beschränkt ist.

Die im Rahmen der vorliegenden Erfindung vorgeschlagene Anordnung der Leuchtmittel in einem Leuchtmittelsegment, mit dem im rechten Winkel und in einem definierten Abstand dazu angeordneten trichterförmigen Reflektor erzielt eine gegenüber dem Stand der Technik vielfach höhere Bestrahlungsstärke und Bestrahlungshomogenität, wobei die einzelnen Leuchtmittel eine relativ geringe Energieaufnahme aufweisen und keine aufwendige Ansteuerung, Kühlung oder Strahlungsschutzmechanismen benötigen.

Wie bereits zuvor beschrieben worden ist, können als Leuchtmittel bevorzugt handelsübliche Leuchtdioden zum Einsatz kommen. Die Auswahl der jeweils verwendeten Leuchtmittel wird hinsichtlich der abgestrahlten spektralen Hauptwellenlängen von der in der jeweiligen Bestrahlungsvorrichtung benötigten effektiven Bestrahlungswellenlänge bestimmt. So können beispielsweise Leuchtdioden mit einer Wellenlänge im Bereich 630 nm, insbesondere im Bereich von 635 nm, verwendet werden, wenn bei einer medizinischen Photodynamischen Therapieanwendung der gewünschte medizinische Effekt dieser Therapie im Bereich dieser Wellenlänge auftritt. Bei einer medizinischen Photodynamischen Diagnoseanwendung können Wellenlängen im Bereich 340 bis 430 nm erforderlich sein, wenn die zur Diagnose notwendige Fluoreszenzanregung beispielsweise bei den Wellenlängen 370 nm bis 428 nm auftritt. Bei einer kosmetischen Anwendung, wie beispielsweise der Haarbleichung können Leuchtmittel verwendet werden, deren emittierte Wellenlänge sich im Bereich von 450 bis 550 nm befindet, wenn der kosmetische Effekt des Haarbleichens in der Nähe dieser Wellenlänge auftritt. Bei einer kosmetischen Anwendung, wie beispielsweise dem Zahnbleichen, können Leuchtmittel verwendet werden, deren emittierte Wellenlänge sich im Bereich von 600 bis 650 nm oder 430 bis 470 nm befindet, wenn der kosmetische Effekt dieser Anwendung in diesem Wellenlängenbereich auftritt. Bei einer industriellen Anwendung, wie beispielsweise dem Härten von Füllstoffen und Klebern, können Leuchtmittel verwendet werden, deren emittierte Wellenlängen sich im Bereich von 430 bis 500 nm befinden, wenn der gewünschte Effekt des Aushärtens in diesem Wellenlängenbereich auftritt.

In Fällen in denen der gewünschte Effekt durch das gleichzeitige Bestrahlen mit weit auseinanderliegenden spektralen Wellenlängenbereichen erzielt wird, kann das benötigte Gesamtemissionsspektrum an der Austrittsöffnung des trichterförmigen Reflektors durch entsprechend definiertes gemischtes, flächiges Anordnen unterschiedlicher Leuchtmittel im selben Leuchtmittelsegment, mit unterschiedlichen Emissionsspektren und deren gleichzeitiges Einstrahlen in den trichterförmigen Reflektor erfolgen.

Die auf der gewünschten Bestrahlungsfläche erzeugte Vervielfachung der Bestrahlungsstärke ist abhängig von der Anzahl und der geometrischen Form der flächig angeordneten Leuchtmittel in einem Leuchtmittelsegment, dessen Positionierung in der Lichteintrittsöffnung des trichterförmigen Reflektors und der Länge sowie der geometrischen Form des verwendeten trichterförmigen Reflektors. Das Leuchtmittelsegment ist vorzugsweise unmittelbar in der Lichteintrittsöffnung derart angeordnet, dass das Leuchtmittelsegment bzw. die effektive geometrische Grundfläche desselben, welche Strahlung abgibt, exakt die Lichteintrittsöffnung abdeckt. Die gewünschte optimale Bestrahlungsstärke wird an der Lichtausgangsöffnung des trichterförmigen Reflektors erzielt. In einem nur um wenige Zentimeter vergrößertem Abstand hinter der Lichtausgangsöffnung des trichterförmigen Reflektors sinkt die Bestrahlungsstärke wieder um ein Vielfaches und gewährleistet so eine maximale Sicherheit des Anwenders oder des Behandelten.

Bei ähnlicher Anordnung unter Verwendung von Laserdioden (statt Leuchtdioden) ist vor die Lichtausgangsöffnung des trichterförmigen Reflektors ein Streumedium zu schalten, um eine entsprechend homogene Lichtverteilung über die Fläche und die erwünschten Sicherheitsmerkmale zu gewährleisten, meist wird hierfür eine sog. Streuscheibe (Glas mit aufgerauter Oberfläche oder aufgebrachter Färbung) verwendet.

Bei Verwendung der vorliegenden Erfindung für schwer zugängliche Bereiche, beispielsweise bei Anwendungen der Photodynamischen Therapie in menschlichen Organen, kann an die Lichtausgangsöffnung des trichterförmigen Reflektors ein Lichtleiter adaptiert werden, der das so verstärkte Licht zum Zielgewebe leitet. Dies ist insbesondere bei der endoskopischen Operationstechnik oder beim Aushärten von Füll- oder Klebstoffen in Hohlkörpern und schlecht zugänglichen Gehäusebereichen notwendig.

Die im Rahmen der vorliegenden Erfindung verwendeten Leuchtmittel können in dem Leuchtmittelsegment insbesondere flächig und gleichmäßig angeordnet sein, vorzugsweise in Reihen und in Spalten. Besonders vorteilhaft ist auch die Anordnung der Leuchtmittel in abwechselnd zueinander versetzten Reihen sowie die Versorgung der Leuchtmittel des Leuchtmittelsegments mit Konstantstrom einer Energieversorgungseinrichtung, wobei im letztgenannten Fall insbesondere die jeweils in einer Reihe angeordneten Leuchtmittel in Serie miteinander verschaltet und mit der Energieversorgungseinrichtung verbunden sind.

Die Leuchtmittel des Leuchtmittelsegments können abhängig von der jeweils gewählten Anwendung Strahlung im ultravioletten Wellenlängenbereich, Strahlung im infraroten Wellenlängenbereich und/oder Strahlung im sichtbaren Wellenlängenbereich etc. erzeugen. Bei einer möglichen Ausführungsform der vorliegenden Erfindung erzeugen sämtliche Leuchtmittel des Leuchtmittelsegments im Wesentlichen Strahlung in ein und demselben Wellenlängenbereich. Ebenso ist es jedoch auch möglich, die Leuchtmittel in dem Leuchtmittelsegment derart in Gruppen zusammenzufassen, dass von Gruppe zu Gruppe Strahlung in unterschiedlichen Wellenlängenbereichen erzeugt wird, um auf diese Weise Strahlungen unterschiedlicher Wellenlängenbereiche miteinander zu mischen und trotzdem eine homogene Bestrahlung zu gewährleisten.

Die Lichteingangsgrundfläche entspricht bevorzugterweise der effektiven, d.h.
Strahlung erzeugenden geometrischen Grundfläche des Leuchtmittelsegments. Die Lichteingangsgrundfläche und die Lichtausgangsgrundfläche des trichterförmigen Reflektors können jeweils rund oder mehr- bzw. vieleckig, vorzugsweise quadratisch, ausgebildet sein.

Im Bereich der Lichtaustritts- bzw. Lichtausgangsöffnung des trichterförmigen Reflektors kann ein Lichtleiter angeordnet sein, in den die Strahlung eingekoppelt wird, wobei die Verwendung eines derartigen Lichtleiters besonders vorteilhaft ist, um zur Bestrahlung in Körperhöhlen eindringen zu können.

Die vorliegende Erfindung kann in Bestrahlungssystemen unterschiedlichster Art eingesetzt werden. So ist beispielsweise die Verwendung in einem medizinischen photodynamischen Diagnosesystem, einem medizinischen photodynamischen Therapiesystem, einem System zum Bleichen von Zähnen oder zum Bleichen von Haaren, einem System zur lokalen Bestrahlung der menschlichen Haut für kosmetische Zwecke zur Klärung von Hautunreinheiten und Pickeln ohne gleichzeitige Anwendung von Medikamenten (wie in der photodynamischen Therapie üblich), einem System zum Härten von Klebern, Lacken, Füllstoffen und anderen chemischen Substanzen, welche mit Licht definierter Wellenlängenbereiche reagieren, oder einem System zur Bestrahlung gleichzeitig mehrerer Flächen von Körpern möglich. Ebenso kann die erfindungsgemäße Bestrahlungsvorrichtung auf einem ferngesteuerten Arm montiert sein, der eine definierte größere Fläche innerhalb einer bestimmten Zeit komplett oder in Teilen abfährt, wie es insbesondere bei so genannten Scannern üblich ist. Auch die Anwendung in Kombination mit einem Endoskop ist denkbar, wobei in diesem Fall der zuvor erwähnte Lichtleiter in den Arbeitskanal des Endoskops eingeschoben werden kann, um den Lichtleiter zur minimal invasiven Behandlung von Körperorganen zu benutzen.

Der trichterförmige Reflektor der erfindungsgemäßen Bestrahlungsvorrichtung kann sowohl als Hohlkörper, bei dem an den Innenflächen des trichterförmigen Reflektors die von dem Leuchtmittelsegment erzeugte Strahlung reflektiert wird, als auch als Voll-Massivkörper aus einem strahlungsdurchlässigen oder strahlungsführenden Material, insbesondere Glas oder Kunststoff (vorzugsweise PMMA-Kunststoff), wobei die Reflexion der Strahlung durch Totalreflexion an den Außen- bzw. Seitenflächen des Voll-Massivkörpers erfolgt, ausgestaltet sein.

Es werden nachfolgend unter Bezugnahme auf die beigefügte Zeichnung der grundsätzliche Strahlungsverlauf in einer erfindungsgemäßen Bestrahlungsvorrichtung und unterschiedliche Ausführungsbeispiele einer erfindungsgemäßen Bestrahlungsvorrichtung bzw. eines erfindungsgemäßen Bestrahlungssystems erläutert, welche beispielsweise zur medizinischen Photodynamischen Diagnostik und Therapie von kleinen Gewebeoberflächen, zum Bestrahlen von kleinen Hautarealen insbesondere zur Behandlung der Akne, zum Auslösen lichtinduzierter Reaktionen in und mit chemischen Stoffen, wie Bleichen von Zähnen und Haaren sowie das Härten von industriellen und zahnmedizinischen Füll- und Klebstoffen sowie Lacken geeignet sind. Selbstverständlich ist die vorliegende Erfindung nicht auf die nachfolgend näher beschriebenen Anwendungsbeispiele beschränkt, sondern kann allgemein überall dort zur Anwendung kommen, wo mit einfachem Aufwand die Erzielung einer hohen Bestrahlungsstärke auf einer kleinen Bestrahlungsfläche erwünscht ist.

### Erläuterung der Figuren

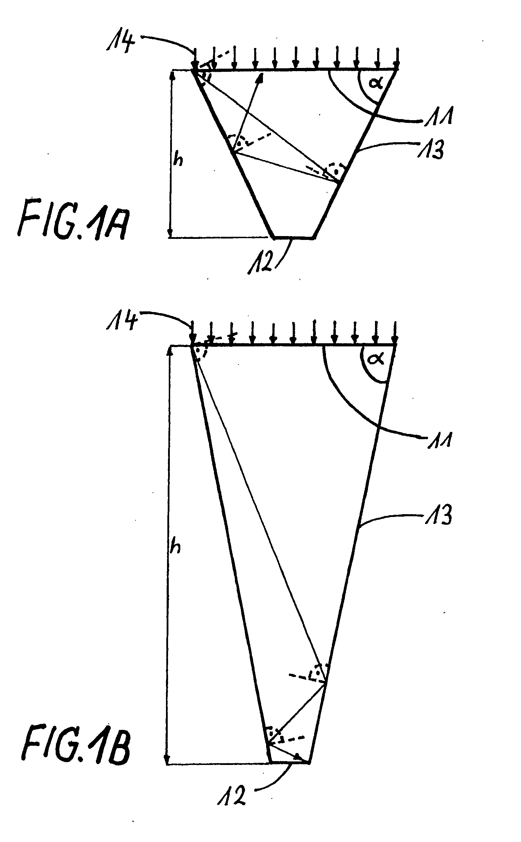

Figur 1A und Figur 1B erläutern als Schnittzeichnungen anhand von zwei Reflektortrichtern (13) mit unterschiedlicher Höhe (h) und unterschiedlichem Öffnungswinkel (α) den grundsätzlichen Verlauf der unterschiedlichen Strahlengänge der jeweils äußersten Randstrahlen (14) der Leuchtmittelsegmente (11) durch den jeweiligen Reflektortrichter (13). Dabei zeigt sich, dass bei vorgegebener Größe von Leuchtmittelsegment (11) und Bestrahlungsfläche (12) nur bei ausreichend großem Öffnungswinkel (α), wie in Figur1B dargestellt, alle Randstrahlen nach mehreren Reflexionen in dem Reflektortrichter (13) an der Lichtausgangsöffnung auf die Bestrahlungsfläche (12) auftreffen, während ein Großteil der Randstrahlen bei nicht ausreichend großem Öffnungswinkel (α), wie in Figur1A dargestellt, nach mehreren Reflexionen in das Leuchtmittelsegmente (11) zurück reflektiert werden und somit nicht wie gewünscht, auf die Bestrahlungsfläche (12) auftreffen. Mit der Bedingung, dass der äusserste Randstrahl (14) aus der Lichtausgangsöffnung austritt und auf die Bestrahlungsfläche (12) trifft ist sichergestellt, dass alle von dem Leuchtmittelsegment (11) weiter innen ausgesandten Strahlen ebenso auf die Bestrahlungsfläche (12) auftreffen. Es zeigt sich somit, dass mit der hier vorgestellten erfindungsgemäßen Bestrahlungsvorrichtung mit dem Reflektortrichter (13), dessen Öffnungswinkel (α), wie in Figur 1B gezeigt, mindestens so groß ist, dass der äußerste Randstrahl des Leuchtmittelsegments (11) noch aus der Lichtausgangsöffnung austreten kann, mit handelsüblichen Leuchtdioden als Leuchtmittel eine sehr hohe Bestrahlungsstärke auf der Bestrahlungsfläche (12) direkt an der Lichtausgangsöffnung erzielt wird.

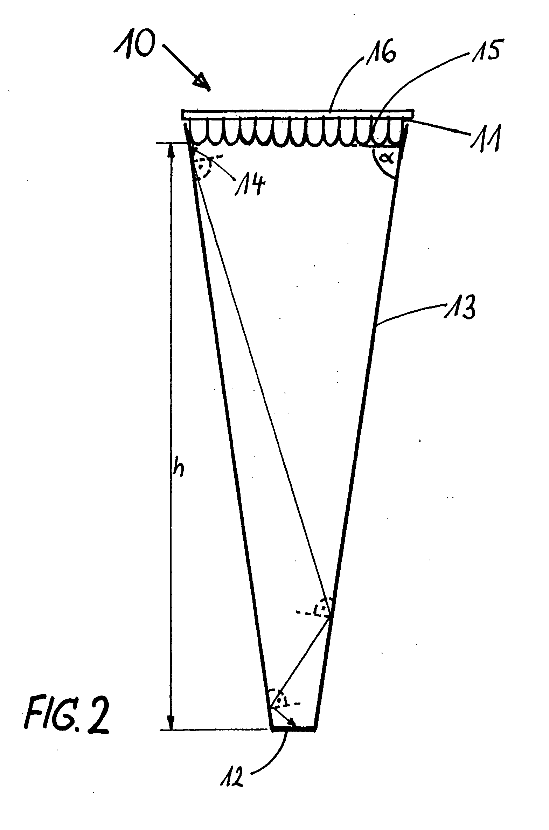

Figur 2 zeigt als Schnittzeichnung eine erfindungsgemäße Bestrahlungsvorrichtung (10) mit dem Reflektortrichter (13) und mit der auf einer Leiterplatte (16) montierten Leuchtdiodenmatrix (15) als Leuchtmittelsegment (11) und dem Randstrahl (14) der äußersten Leuchtdiode, dabei wurde bei vorgegebener Größe von Leuchtmittelsegment (11) und Lichtausgangsöffnung bzw. Bestrahlungsfläche (12) der Öffnungswinkel (α) noch etwas größer als der Mindestöffnungswinkel gewählt, um Toleranzen sicher auszugleichen.

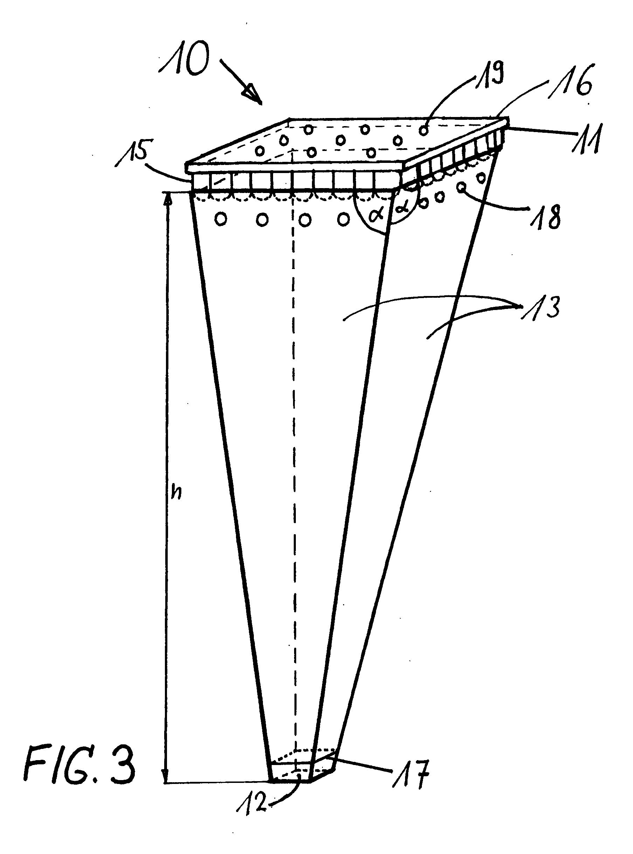

Figur 3 zeigt als Ansicht eine erfindungsgemäße Bestrahlungsvorrichtung (10) mit einem Reflektortrichter (13) in Form eines Pyramidenstumpfes und dem Leuchtmittelsegment (11) bestehend aus einer auf einer Leiterplatte (16) bestückten Leuchtdiodenmatrix (15), welches zur besseren optischen Einkopplung in den Reflektortrichter vertieft angeordnet ist. Direkt an der Austrittsöffnung wird die von dem Bestrahlungselement (11) abgestrahlte Strahlung auf die kleine Bestrahlungsfläche (12) fokussiert, und erreicht dort die für die Bestrahlung erforderliche sehr hohe Bestrahlungsstärke (mW/qcm). Schon in wenigen cm Abstand von der Austrittsöffnung streut die Strahlung so stark auseinander, dass keine Personengefährdung mehr besteht. Vorteilhaft zum Abschluss der Öffnung ist eine Verschlussplatte (17) aus einem strahlungsdurchlässigen Material vorzugsweise Acrylglas an der Lichtausgangsöffnung angeordnet. Ebenso vorteilhaft ist bei max. Leistung der Leuchtdioden oder Laserdioden eine Kühlung des Leuchtmittelsegments (11), welche durch einen hinter der Leiterplatte angeordneten Ventilator (19) bewerkstelligt wird, der die Kühlluft von außen durch den mit Löcher (18) versehenen Reflektortrichter (13) ansaugt und zwischen den zu kühlenden Leuchtdioden (15) hindurch durch weitere Kühllöcher (19) in der Leiterplatte (16) nach außen absaugt.

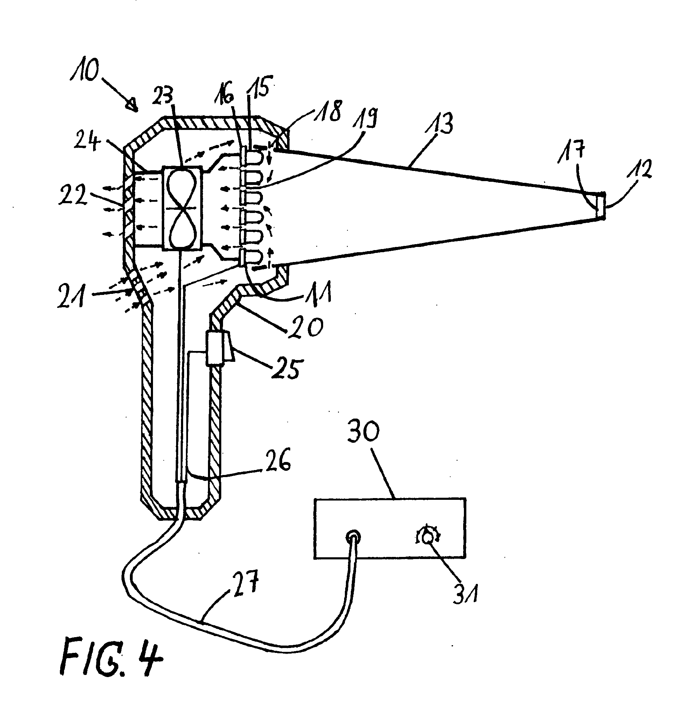

Figur 4 zeigt als Querschnittzeichnung eine erfindungsgemäße Bestrahlungsvorrichtung (10) in einer Anwendung als tragbares Handbestrahlungssystem zur medizinischen Behandlung von erkrankten Hautpartien z.B. bei Akne oder zur Photodynamischen Therapie PDT oder zur kosmetischen Behandlung z.B. zur Bleichung von Zähnen oder zur Bleichung von Haaren oder zum industriellen Aushärten z.B. von Klebern, Vergußmassen oder Lacken. Der Reflektortrichter (13) mit dem aus der Leiterplatte (16) und den darauf montierten Leuchtdioden (15) bestehenden Leuchtmittelsegment (11) ist in dem Gehäuse (20) des im unteren Teil als Handgriff geformten Bestrahlungssystems fest fixiert. Die strahlungsdurchlässige Verschlussplatte (17) an der Lichtausgangsöffnung erlaubt beispielsweise das Bestrahlungsystem auf die Hautpartie aufzusetzen, sodass die Bestrahlungsfläche (12) direkt auf der Haut liegt. Die bei hoher Leuchtmittelleistung vorteilhafte Kühlung des Leuchtmittelsegmentes (11) ist mit gestrichelten Pfeilen dargestellt, wobei der hinter dem Leuchtmittelsegment (11) angeordnete Ventilator (23) die Kühlluft von außen durch die Zuluftkühlrippen (21) dann durch die Löcher (18) im Reflektortrichter (13) und weiter zwischen den zu kühlenden Leuchtdioden (15) durch die Leiterplattenkühllöcher (19) und die saugseitige Hutze (24) ansaugt, und die erwärmte Abluft durch die druckseitige Hutze (24) und die Abluftkühlrippen (22) nach außen ausbläst. Das Steuerteil (30) beinhaltet die elektrische Steuerung für das Handbestrahlungssystem beispielsweise für das Leuchtmittelsegment (11), den Ventilator und die Bestrahlungszeit, die beispielsweise dem Knopf (31) am Steuerteil (30) einstellbar ist und mit dem Schalter (25) am Handgriff gestartet wird. Mit dem Steuerteil verbunden sind die einzelnen Komponenten des Handteils mittels der internen Verdrahtung (26) und der Zuleitung (27). Selbstverständlich kann die von dem Ventilator (23) erzeugte Kühlluftventilation auch in umgekehrter Richtung erfolgen.

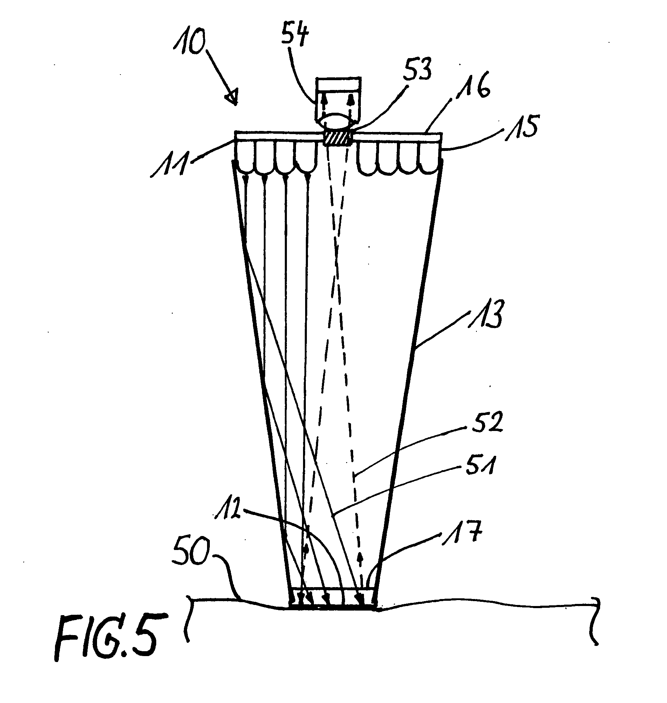

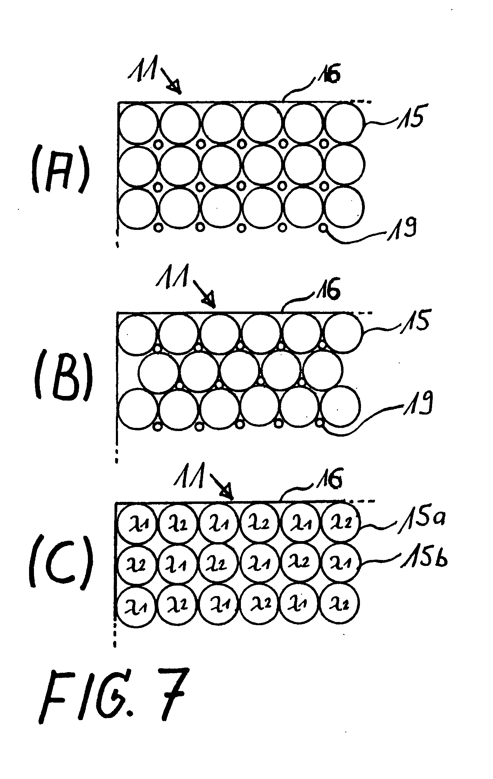

Figur 5 zeigt als Schnittzeichnung eine erfindungsgemäße Bestrahlungsvorrichtung zur medizinischen Photodynamischen Diagnose (PDD), wobei hier der Grundaufbau im wesentlichen der in Figur2 dargestellten Bestrahlungsvorrichtung entspricht. Die Bestrahlungvorrichtung (10) liegt mit der strahlungsdurchlässigen Verschlußplatte (17) des Reflektortrichters (13) auf der Patientenhautpartie (50) auf, sodass die Bestrahlungsfläche (12) direkt auf der Haut liegt. Das aus der Leiterplatte (16) und den Leuchtmittel (15) bestehende Leuchtmittelsegment (11) sendet kurzwellige Strahlung beispielsweise im Bereich zwischen 370nm und 430nm als Anregungsstrahlung aus, wobei hier beispielsweise unterschiedliche Leuchtmittel mit verschiedenen Hauptwellenlängen λ1 und λ2 (siehe auch Figur7c) verwendet werden können. Die fokussierte Anregungsstrahlung (51) ruft in Abhängigkeit von einer vorher dem Patienten verabreichten Substanz bei krankhaft veränderten Hautpartien, wie beispielsweise Tumoren eine signifikant verstärkte langwellige Fluoreszenzstrahlung (52) beispielsweise im Bereich 600nm bis 700nm hervor. In dem Leuchtmittelsegment (11) befindet sich eine Öffnung vorzugsweise mit einem optischen Filter (53) mit einem Transmissionsbereich im Bereich der Fluoreszenzstrahlung und einem optischen Auswertesystem (54) beispielsweise einer Kamera, mit dem eventuelle Veränderungen der krankhaften Hautpartien diagnostiziert und ausgewertet werden. In der Figur5 sind beispielhaft einige Anregungsstrahlen (51) als Vollinien und einige Fluoreszenzstrahlen (52) als gestrichelte Linien dargestellt.

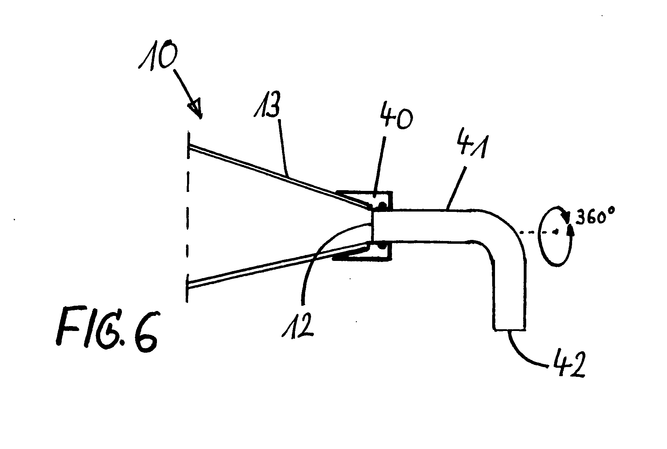

Figur 6 zeigt als Schnittzeichnungsdetail den vorderen Abschnitt einer erfindungsgemäßen Bestrahlungsvorrichtung (10) mit einer im Bereich der Lichtausgangsöffnung angebrachten Lichtleiterhalterung (40) und einem strahlungsdurchlässigen Lichtleiter (41), beispielsweise zur therapeutischen Anwendung mit Endoskopen, wobei hier der Grundaufbau im wesentlichen der in Figur2 dargestellten Bestrahlungsvorrichtung entspricht. Jedoch ist hier an der Lichtausgangsöffnung nicht das Bestrahlungsobjekt selbst zu finden, sondern die Einkopplungsfläche eines Lichtleiters, d.h. die Bestrahlungsfläche (12) liegt auf der Lichtleitereinkopplungsfläche und die vom Reflektortrichter (13) auf die Bestrahlungsfläche (12) fokussierte Strahlung wird direkt in den Lichtleiter eingekoppelt und von diesem an die Lichtleiterauskopplungsfläche (42) weitergeleitet, wo sich das vorgesehene Bestrahlungsobjekt befindet. Vorzugsweise ermöglicht die Lichtleiterhalterung (40) eine feste Fixierung des Lichtleiters bei voller Drehbarkeit um 360 Grad. Da der Lichleiter normalerweise rund ist, ist der Reflektortrichter und das Leuchtmittelsegment in dieser Anwendung vorzugsweise auch rund d.h. der Reflektortrichter (13) ist als Kegelstumpf ausgebildet. wobei die gleichen Strahlungsbedingungen wie bei der eckigen Ausführung gelten. Anstatt eines starren Lichtleiters kann auch ein flexibler Lichtleiter eingesetzt werden.

Figur 7 zeigt als prinzipielle Darstellung verschiedene Anordnungen von Leuchtmittel (15), vorzugsweise Leuchtdioden oder Laserdioden auf der Trägerleiterplatte (16) eines Leuchtmittelsegments (11) einer erfindungsgemäßen Bestrahlungsvorrichtung.
Figur 7A zeigt eine Anordnung der Leuchtmittel (15) in geraden Reihen, wobei zwischen den Leuchtmittel (15) nicht unbedingt, jedoch vorteilhaft vorzugsweise bei hohen Diodenleistungen Kühlluftbohrungen (19) in der Leiterplatte (16) angebracht sind.
Figur 7B zeigt eine Anordnung der Leuchtmittel (15) in versetzten Reihen, wobei zwischen den Leuchtmittel (15) nicht unbedingt, jedoch vorteilhaft vorzugsweise bei hohen Diodenleistungen Kühlluftbohrungen (19) in der Leiterplatte (16) angebracht sind. Versetzte Reihen ermöglichen auf gleicher Leuchtmittelsegmentfläche mehr Leuchtmittel zu plazieren als mit geraden Reihen, und so die erzielbare Bestrahlungsstärke auf der jeweiligen Bestrahlungsfläche noch weiter zu erhöhen.
Figur 7C zeigt beispielhaft eine Anordnung von Leuchtmittel (15a) und (15b) mit jeweils unterschiedlicher Strahlung der Hauptwellenlänge λ1 und Hauptwellenlänge λ2, beispielsweise mit λ1=370nm und λ2=428nm für eine breitbandige Anregungsstrahlung (siehe auch Figur5). Auch kann beispielsweise abwechselnd die eine oder die andere Strahlungswellenlänge in einer Anwendung vorteilhaft ermöglicht werden. Es können auch mehr als nur zwei unterschiedliche Leuchtmittel in einem Leuchtmittelsegment angeordnet werden um mehr als nur zwei Strahlungswellenlängen zusammen oder einzeln anzuwenden.

## Patentansprüche

1. Bestrahlungsvorrichtung,
mit einem trichterförmigen Reflektor (13), welcher eine Lichteingangsgrundfläche und eine Lichtausgangsgrundfläche aufweist, wobei die Form der Lichtausgangsgrundfläche zwar der Form der Lichteingangsgrundfläche entspricht, die Lichtausgangsgrundfläche jedoch kleiner als die Lichteingangsgrundfläche ist und einer gewünschten definierten Bestrahlungsfläche (12) entspricht,
**dadurch gekennzeichnet,**
**dass** im Bereich der Lichteingangsgrundfläche des trichterförmigen Reflektors (13) mindestens ein flächiges Leuchtmittelsegment (11) angeordnet ist, wobei
das Leuchtmittelsegment (11) einen Träger (16) umfasst, auf dem eine Vielzahl von Leuchtmitteln (15) derart angeordnet ist, dass eine lichtabstrahlende geometrische Grundfläche des Leuchtmittelsegments (11) in seiner Form im Wesentlichen der Lichteingangsgrundfläche des trichterförmigen Reflektors (13) entspricht, und
**dass** ein Öffnungswinkel (α) des trichterförmigen Reflektors (13) bei vorgegebener Größe des Leuchtmittelsegments (11) und der Bestrahlungsfläche (12) mindestens so groß ist, dass auch ein von dem Leuchtmittelsegment (11) erzeugter äußerster Randstrahl (14) gerade noch auf die Bestrahlungsfläche (12) trifft.

2. Bestrahlungsvorrichtung nach Anspruch 1,
**dadurch gekennzeichnet,**
**dass** das Leuchtmittelsegment (11) eine Vielzahl von flächig, gleichmäßig angeordneten Leuchtmittel (15) umfasst.

3. Bestrahlungsvorrichtung nach Anspruch 1 oder 2,
**dadurch gekennzeichnet,**
**dass** die Leuchtmittel (15) Leuchtdioden oder Laserdioden sind, deren Abstrahlwinkel jeweils maximal 30° beträgt.

4. Bestrahlungsvorrichtung nach einem der vorhergehenden Ansprüche,
**dadurch gekennzeichnet,**
**dass** die Leuchtmittel (15) des Leuchtmittelsegments (11) auf dem Träger (16) in mehreren Gruppen (15a, 15b) angeordnet sind, wobei die Leuchtmittel (15) jeder Gruppe (15a, 15b) Strahlung in einem unterschiedlichen Wellenlängenbereich erzeugen.

5. Bestrahlungsvorrichtung nach einem der vorhergehenden Ansprüche,
**dadurch gekennzeichnet,**
**dass** die geometrische Grundfläche des Leuchtmittelsegments (11), die Lichteingangsgrundfläche und die Lichtausgangsgrundfläche des trichterförmigen Reflektors (13) rund oder mehreckig ausgebildet sind.

6. Bestrahlungsvorrichtung nach einem der vorhergehenden Ansprüche,
**dadurch gekennzeichnet,**
**dass** das Leuchtmittelsegment (11) und der trichterförmige Reflektor (13) in einem tragbaren Gehäuse (20) untergebracht sind.

7. Bestrahlungsvorrichtung nach einem der vorhergehenden Ansprüche,
**dadurch gekennzeichnet,**
**dass** die Bestrahlungsvorrichtung eine Detektoreinrichtung (54) zum Erfassen von an der Bestrahlungsfläche (12) in Folge der Bestrahlung durch die Strahlungen des Leuchtmittelsegments (11) angeregte Fluoreszenzstrahlung (52) umfasst.

8. Bestrahlungsvorrichtung nach Anspruch 7,
**dadurch gekennzeichnet,**
**dass** die Detektoreinrichtung (54) ein optisches Filterelement (53) mit einem der angeregten Fluoreszenzstrahlung (52) entsprechenden Durchlassbereich umfasst.

9. Bestrahlungsvorrichtung nach einem der vorhergehenden Ansprüche,
**dadurch gekennzeichnet,**
**dass** die Bestrahlungsvorrichtung in einem mobilen Gehäuse zur Positionierung der Bestrahlungsvorrichtung über einem gewünschten Körperbereich untergebracht ist.

10. Bestrahlungsvorrichtung nach einem der vorhergehenden Ansprüche,
**dadurch gekennzeichnet,**
**dass** die Bestrahlungsvorrichtung im Bereich des Lichtausgangs des trichterförmigen Reflektors (13) einen Lichtleiter (41) besitzt, in den das Licht von der Lichtausgangsgrundfläche eingekoppelt wird.

11. Bestrahlungsvorrichtung nach Anspruch 10,
**dadurch gekennzeichnet,**
**dass** dieser Lichtleiter (41) gekröpft ist.

12. Bestrahlungsvorrichtung nach Anspruch 10 oder 11,
**dadurch gekennzeichnet,**
**dass** dieser Lichtleiter (41) im rechten Winkel gebogen ist.

13. Bestrahlungsvorrichtung nach einem der Ansprüche 10-12,
**dadurch gekennzeichnet,**
**dass** der Lichtleiter (41) in einer Fassung (40) drehbar angebracht ist und in verschiedenen Stellungen innerhalb von 360° fixierbar ist.

14. Bestrahlungsvorrichtung nach einem der vorhergehenden Ansprüche,
**dadurch gekennzeichnet,**
**dass** die Bestrahlungsvorrichtung auf einem ferngesteuerten Arm montiert ist, der eine definierte grössere Fläche innerhalb einer bestimmten Zeit komplett oder in Teilen abfährt.

15. Bestrahlungsvorrichtung nach einem der vorhergehenden Ansprüche,
**dadurch gekennzeichnet,**
**dass** die Bestrahlungsvorrichtung ein Luftkühlsystem enthält, welches zur Kühlung eine Ventilation von Kühlluft durch mindestens eine im trichterförmigen Reflektor (13) ausgebildete Öffnung (18) oder durch einen Spalt zwischen dem Leuchtmittelsegment (11) und dem trichterförmigen Reflektor (13) und durch in dem Träger (16) ausgebildete Löcher (19) an den Leuchtmitteln (15) vorbei erzeugt.

16. Bestrahlungsvorrichtung nach Anspruch 15,
**dadurch gekennzeichnet,**
**dass** das Lüftkühlsystem einen mit dem Leuchtmittelsegment (11) in einem Gehäuse (20) angeordneten Ventilator (23) umfasst,
wobei der Ventilator (23) benachbart zu dem Leuchtmittelsegment (11) derart positioniert ist, dass der Ventilator (23) die Kühlluft von außen durch eine Gehäuseöffnung (21) durch die mindestens eine in den trichterförmigen Reflektor (13) ausgebildete Öffnung (18) oder durch den Spalt zwischen dem Leuchtmittelsegment (11) und dem trichterförmigen Reflektor (13), an den Leuchtmitteln (15) vorbei durch die Löcher (19) in den Träger (16) und über eine weitere Gehäuseöffnung (22) nach außen oder in umgekehrter Richtung bewegt.

17. Bestrahlungsvorrichtung nach einem der vorhergehenden Ansprüche,
**dadurch gekennzeichnet,**
**dass** der Lichtausgang des trichterförmigen Reflektors (13) mit einem die Strahlung des Leuchtmittelsegments (11) durchlassenden und gegen Eindringen von Feuchtigkeit abgedichteten Verschlusselement (17) ausgerüstet ist.

18. Bestrahlungsvorrichtung nach Anspruch 17,
**dadurch gekennzeichnet,**
**dass** dieses Verschlusselement (17) mit einem den entsprechenden Wellenlängenbereich der Strahlung durchlassenden überstülpbaren Folienkäppchen in Form eines sterilen Einmalartikels für den Haut- oder Gewebekontakt ausgestattet ist.

19. Bestrahlungsvorrichtung nach einem der vorhergehenden Ansprüche,
**dadurch gekennzeichnet,**
**dass** der trichterförmige Reflektor (13) als Voll-Massivkörper aus einem strahlungsleitenden Material ausgeführt ist, bei dem eine Reflexion der von dem Leuchtmittelsegment (11) erzeugten Strahlung durch Totalreflexion an Seitenflächen des Voll-Massivkörpers erfolgt.

20. Medizinisches photodynamisches Diagnosesystem, medizinisches photodynamisches Therapiesystem, System zum Bleichen von Zähnen oder Haaren, System zur lokalen Bestrahlung menschlicher Haut für kosmetische Zwecke zur Klärung von Hautunreinheiten ohne gleichzeitige Anwendung von Medikamenten, System zum Härten einer chemischen Substanz, System zur gleichzeitigen Bestrahlung mehrerer Flächen eines Körpers oder Endoskop mit einer Bestrahlungsvorrichtung nach einem der vorhergehenden Ansprüche.

21. Endoskop nach Anspruch 20,
**dadurch gekennzeichnet,**
**dass** die Bestrahlungsvorrichtung eine Bestrahlungsvorrichtung mit einem Lichtleiter (41) nach einem der Ansprüche 10-13 ist,
wobei der Lichtleiter in einen Arbeitskanal des Endoskops zur minimal invasiven Behandlung von Körperorganen eingeschoben ist.
